# EUROPEAN PATENT APPLICATION

(11) **EP 1 764 610 A1**
(43) Date of publication of application: **21.03.2007**
(21) Application number: 05255710.5
(22) Date of filing: 14.09.2005
(51) Int. Cl.: G01N 21/87, G02B 21/26, G01N 33/38

(54) **Improvements in gemstone viewing methods and apparatus**

(71) Applicant: Overseas Diamonds Technologies N.V., 2018 Antwerpen (BE)
(72) Inventor: Van de Velde, Marc Frans Alida, Suite 803 2018 Antwerpen (BE); Keersmaekers, Christiaan Louis Cecile, Suite 803 2018 Antwerpen (BE)
(74) Representative: Musker, David Charles

(57) **Abstract**

Viewing apparatus for viewing a surface of a gemstone comprising an adjustable platform with a surface adapted to receive the gemstone, a viewing axis along which the gemstone is viewed, and a light source adapted to emit light substantially parallel to the viewing axis, the viewing apparatus characterised by: an alignment means comprising a plurality of misalignment indicators, the misalignment indicators arranged to provide three or more visually distinguishable zones around the viewing axis, the alignment means further comprising three or more adjustment indicators positioned relative to the platform and the misalignment indicators, wherein the adjustment indicators are associated with the visually distinguishable zones such that an image viewed along the viewing axis of one of the zones reflected off the surface of the gemstone provides an indication of which way to adjust the platform and the viewing axis relative to each other to make the surface of the gemstone and the viewing axis perpendicular to each other.

## Description

The present invention relates to improvements in gemstone viewing methods and apparatus. In particular, the present invention relates to an improved method and apparatus for viewing a surface of a gemstone by providing an indication of the required adjustment of the apparatus to enable the surface to be viewed.

To assure the quality of gemstones and the integrity of gemstone manufacturers, gemstones (for example, diamonds) can have quality marks, identification (ID) numbers or brand names ionised on them (herein called collectively 'marks'). Any combination of these marks may be placed onto a surface of the gemstone. In this way, a potential purchaser of a gemstone is able to observe the mark on the gemstone, and use that information to ascertain the origin of the gemstone. This information enables the potential purchaser to make a judgement as to whether or not to purchase the gemstone.

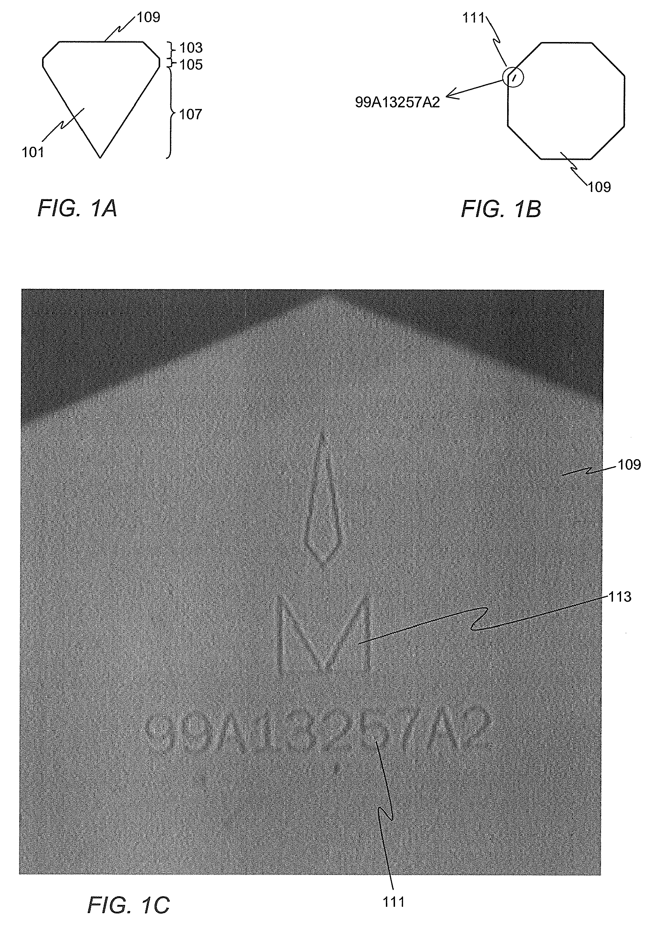

An ideal cut diamond 101 is depicted in Figure 1. The diamond 101 is made up of a crown 103, a girdle 105 and a pavilion 107. The top part of the crown 103 is the table 109.

The mark is ionised, or possibly etched, onto any suitable surface of the gemstone. In the case of an ideal cut diamond, the ionised mark is usually located in one of the eight corners of the diamond's table. Figure 1B shows a pictorial representation of a table 109 of an ideal cut diamond with an ionised ID number 111. Figure 1C shows a real image of a corner of a table 109 of an ideal cut diamond with an ionised ID number 111 and company logo 113.

As an alternative to placing the ionised mark on the table of a gemstone, it is also possible to place the ionised mark on any other relatively flat surface of a gemstone. For example, the ionised mark may be placed on the girdle or one of the facets of the gemstone.

The size of the mark relative to the gemstone table is very small, in order to ensure the mark is not visible to the naked eye. For example, an ID number consisting of 10 characters is usually in the order of 0.3 mm in horizontal length. Whereas, 0.5 ct, 1 ct and 2 ct ideal cut diamonds have a table size in the order of 2.9mm, 3.7mm and 4.7mm diameter, respectively. Therefore it can be seen that a 10 character ID number is approximately 6% - 10 % the size of the table of an ideal cut diamond.

There are several well-known methods currently used to view an ionised mark situated on a gemstone surface.

In order to view a mark on the gemstone surface, the person viewing the gemstone must try and arrange the surface on to which the mark has been ionised, for example the table, to reflect any available light source off the surface directly towards their eye. When the table is positioned correctly, retro-reflection occurs. This makes the table appear bright white, while the ionised mark on the table appears as a darker grey colour due to the relatively uneven ionised surface causing the light in that area to be reflected off it in many different directions.

One method used to view a mark on a gemstone is to hold the gemstone with tweezers, or any other suitable handling device, whilst attempting to view the mark through a jeweller's high magnification loupe with a light source directed towards the ionised surface. When carrying out this method, it is very difficult to hold the gemstone steady in order to read the mark, as well as position the surface at the correct angle in relation to the light source in order to cause retro-reflection to occur.

In an alternative known method, International Patent application WO 99/34197 by Gersan Establishment discusses a viewing device used to view marks on a gemstone. This device has two modes. In a first mode, the gemstone is viewed with diffused light in order to adjust the position of the gemstone to allow it to be viewed on a display monitor using a camera. In this first mode, the viewer can inspect the workmanship of the gemstone, but is unable to view the mark. In a second mode, the gemstone is viewed through a pinhole aperture, and the table upon which the gemstone is situated may be linearly adjusted or tilted in order to cause retro reflection to occur, and so view the mark.

Also, viewing equipment is available to enable a user to view a surface of a gemstone to determine if there are any defects or anomalies upon the surface. For example, surface smoothness (finish) can be assessed. Again, retro-reflection is required to enable the surface to be viewed by the user of the equipment. However, known viewing equipment does not include any means to show the user how to adjust the viewing equipment in order to cause retro-reflection.

In all the above-described known methods and systems there is no provision to enable a user to determine in which direction the surface of the gemstone must be moved or tilted in order to cause retro-reflection. Therefore, continual movement with trial and error is required before eventually being able to cause retro-reflection and so enable the mark, defect or anomaly to be viewed successfully.

The present invention aims to overcome, or at least alleviate, some or all of the above-mentioned problems.

In one aspect, the present invention provides viewing apparatus for viewing a surface of a gemstone comprising an adjustable platform with a surface adapted to receive the gemstone, a viewing axis along which the gemstone is viewed, and a light source adapted to emit light substantially parallel to the viewing axis, the viewing apparatus characterised by: an alignment means comprising a plurality of misalignment indicators, the misalignment indicators arranged to provide three or more visually distinguishable zones around the viewing axis, the alignment means further comprising three or more adjustment indicators positioned relative to the platform and the misalignment indicators, wherein the adjustment indicators are associated with the visually distinguishable zones such that an image viewed along the viewing axis of one of the zones reflected off the surface of the gemstone provides an indication of which way to adjust the platform and the viewing axis relative to each other to make the surface of the gemstone and the viewing axis perpendicular to each other.

In a further aspect, the present invention provides a method of viewing a mark on a surface of a gemstone, comprising the steps of: placing the gemstone on a surface of a platform, viewing the gemstone along a viewing axis, the method characterised by the steps of: viewing along the viewing axis a visually distinguishable zone reflecting off the surface of the gemstone, the visually distinguishable zone being associated with an adjustment indicator, and adjusting the platform and the viewing axis relative to each other depending on the associated adjustment indicator.

The present invention provides the advantage of giving a user an indication as to which way to adjust a platform upon which a gemstone is located and a viewing axis relative to each other in order to cause retro-reflection to occur. In this manner, a user is able to view a mark on a gemstone in an easy and efficient manner.

Specific embodiments of the present invention will now be described by way of example only, with reference to the accompanying drawings, in which:
Figure 1A shows the different parts of an ideal cut diamond;
Figure 1B shows a pictorial representation of a diamond table with an ionised ID number;
Figure 1C shows an image of an actual ID number and brand name ionised on a table of a diamond;
Figure 2 shows the concept of retro-reflection as used according to a first embodiment of the present invention;
Figure 3 shows how a set diamond affects retro reflection according to a first embodiment of the present invention;
Figure 4 shows a gemstone viewing device according to a first embodiment of the present invention;
Figure 5A shows a top view of a plexi-glass device used in the first embodiment;
Figure 5B shows a side view of a plexi-glass device used in the first embodiment;
Figure 6A shows a top view of a gemstone viewing device according to the first embodiment;
Figure 6B shows a partial cross section of the view in Figure 6A;
Figure 6C shows an internal view of an eyepiece according to a further embodiment of the present invention;
Figure 7 shows the viewer of the first embodiment in operation;
Figure 8A shows a first example of the concept of providing an indication of which way to adjust a diamond's position according to the first embodiment of the present invention;
Figure 8B shows a second example of the concept of providing an indication of which way to adjust a diamond's position according to the first embodiment of the present invention;
Figure 9 shows the concept of using four colour zones to provide an indication of which way to adjust a diamond's position according to the first embodiment of the present invention.

### PREFERRED EMBODIMENT

In the preferred embodiment described below, reference is made to the gemstone being a diamond. However, it will be understood that the invention may be applied to the viewing of a surface on any gemstone.

Further, in the preferred embodiment described below, the viewing equipment and method is concerned with viewing a mark on a gemstone's surface. However, it will be understood that the equipment and method may also be used to view any defects or anomalies on a gemstone's surface.

Also, in the preferred embodiment described below, the mark is ionised on the table of the gemstone. However, it will be understood that the mark may be ionised on any suitable surface of a gemstone. That is, any surface that is able to receive an ionised mark, which may subsequently be positioned to cause retro reflection to occur.

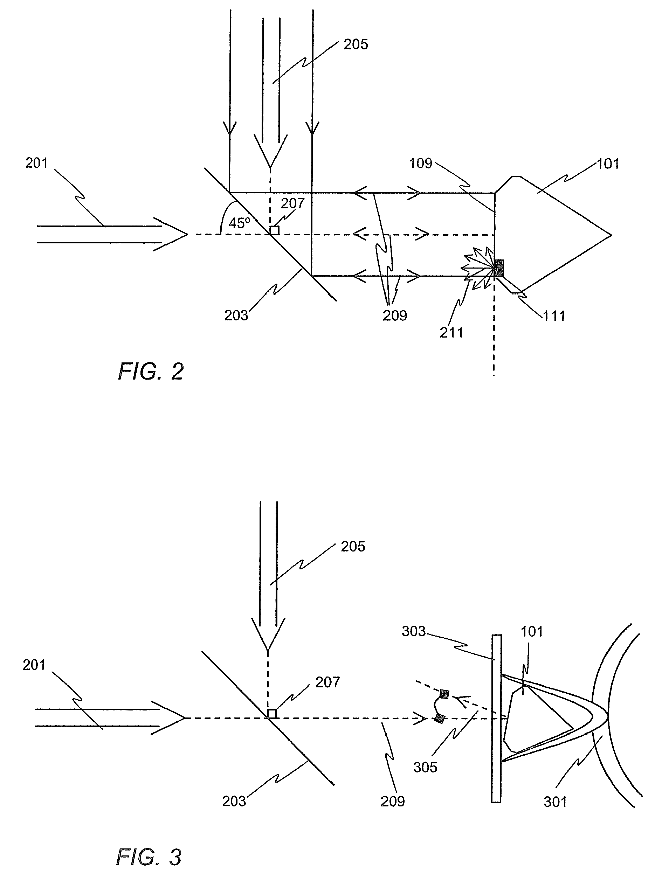

Referring to Figure 2, which shows the concept of retro reflection according to the preferred embodiment described, a diamond 101 includes a table 109 with an ionised mark 111. A user wishing to view the mark 111 looks along a viewing axis 201 towards the diamond 101. The viewing axis passes through partially reflective mirror 203 towards the centre of the table 109 of the diamond 101. The partially reflective mirror 203 works as a beam splitter.

A white light source (not shown) emits white light 205 towards the table 109 of the diamond 101. The white light 205 is directed towards the table 109 by utilising the reflecting surface of the partially reflective mirror 203. The viewing axis 201 and white light propagation direction 205 are perpendicular to each other at the point when the white light 205 has been reflected off the partially reflective mirror 203. The white light and viewing axis 201 become parallel at this point by ensuring the angle 207 between the white light propagation direction and the viewing axis 201 is approximately 90°, while also ensuring that the mirror is set at a 45° angle at the intersection of the white light and viewing axis 201.

When the table 109 of the diamond 101 is arranged to be perpendicular to the viewing axis 201, and so also perpendicular to the white light propagation direction 205, retro reflection occurs. That is, the white light 205 is reflected back along the viewing axis 201 back towards the source, as indicated by the arrows 209.

When retro reflection occurs, the ionised mark 111 on the table 109 of the diamond 101 does not reflect the white light 205 back along the viewing axis 201. Instead, light is reflected off the ionised mark 111 in many different directions as indicated by the arrows 211 in Figure 2. As a large portion of the light reflected off the ionised mark 111 is not reflected back along the viewing axis 201, the ionised mark 111 appears as a light grey colour when compared with the rest of the image of the table 109, thus making it easy to see and read the mark 111.

There are many different ways of setting a diamond within a piece of jewellery, for example, rings, necklaces, bracelets, watches etc. It is often the case that the table of the diamond is located below the top surface of the setting within the piece of jewellery. Figure 3 shows how the setting of a diamond requires the platform to be adjusted in order to make the table of a diamond perpendicular to a viewing axis and light source. A diamond 101 is set in a ring 301. The ring 301 is placed upon a glass platform 303, with the setting resting on the glass platform. The viewing axis 201 passes through the partially reflective mirror 203 towards the centre of the table of the diamond 101. The white light 205 is reflected off the partially reflective mirror 203 at an angle 207 towards the centre of the table of the diamond 101. However, as the table of the diamond is not perpendicular to the viewing axis 201 and the white light 205, retro reflection does not occur. Rather, the white light 205 is passed along the viewing axis 201, as indicated by the arrow 209, towards the table of the diamond 101 and reflected off at an angle 305. It is thus necessary for the user of the viewing device to adjust the position of the glass platform 303 such that the table of the diamond 101 becomes perpendicular with the white light source 205 along the viewing axis 201 in order to cause retro reflection to occur and see the mark. Details of how this adjustment is made are described below.

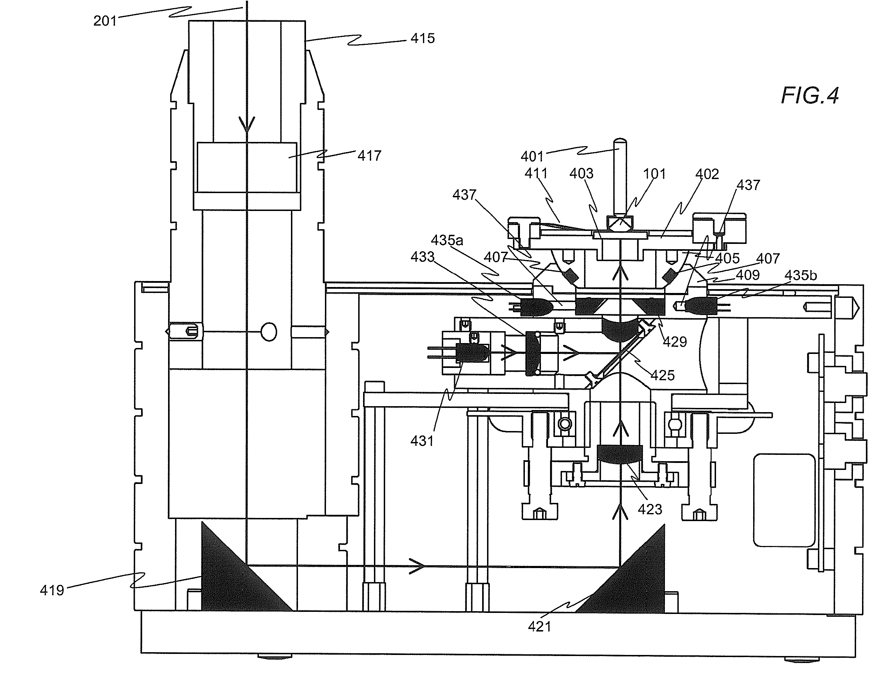

Figure 4 shows a gemstone viewing device according to this specific embodiment. A ring 401 with a diamond 101 is located on a platform 402. The platform 402 includes a glass plate 403, upon which the diamond 101 is placed, with the surface (table) upon which the mark is ionised face downwards, as shown in Figure 4. The ring 401 and diamond 101 are held securely to the surface of the glass plate 403 by a metal blade spring 411. The blade spring 411 includes a rubber outer portion to protect the ring and diamond. The blade spring 411 asserts downward pressure on the ring 401 which holds the diamond 101 to ensure that the ring 401 does not move on the glass plate 403.

The platform 402 also includes a semi spherical platform portion 405 attached to its lower surface. The semi spherical platform portion 405 includes magnetic portions 407. The platform 402, including the semi spherical platform portion 405, is inserted into a metal platform holder 409. In this manner, the platform 402 may be rotated and tilted in any direction thus allowing the surface of the table of the diamond 101 to be tilted and rotated. The magnetic portions 407, when acting with the metal platform holder 409 cause the platform 402 to be fixed in the position it was last moved to.

The platform 402 is tilted and rotated by holding onto the outside edge of the platform 402.

The viewing device includes an eyepiece 415 through which a user may view the table of the diamond 101 along a viewing axis 201. The viewing axis 201 passes through the eyepiece lens 417. In this embodiment, the position of the eyepiece is fixed and the lens has 10x magnification.

The viewing axis 201 is reflected off a first mirror 419 at a 90° angle and is directed towards a second mirror 421, where it is reflected at a 90° angle towards the table of the diamond 101. The viewing axis 201 passes through an objective lens 423, which is a moveable lens for the purposes of focusing. Further, the objective lens 423 provides an optical magnification of 4x. The objective lens may be moved along the viewing axis 201 in order to provide a means to allow a user to focus onto the table of the diamond 101.

After the objective lens 423, the viewing axis 201 passes to a partially reflective mirror 425, which acts as a beam splitter. The partially reflective mirror 425 allows a user to view the diamond 101 along the viewing axis 201. On the reverse side of the partially reflective mirror 425, a reflective coating is placed such that any light directed towards the reflective coating is partially reflected off that surface. However, some of the light is also allowed to pass through the reflecting surface and out the other side of the partially reflective mirror 425 back along the viewing axis 201.

After the viewing axis 201 has passed through the partially reflective mirror 425, it passes through a plexi-glass device 429. The plexi-glass device will be explained in more detail later. The viewing axis 201 continues on to the glass plate 403 located on the platform 402 such that the viewer looking through the eyepiece 415 may view the table of the diamond 101.

Within the viewing device is located a white light source 431, which in this embodiment is a white LED. The LED 431 is connected to a suitable power supply that is operated by an ON/OFF switch (not shown). The white LED 431 emits a white light towards the partially reflective mirror 425. A condenser lens 433 is used to create a parallel beam of white light that covers a larger portion of the partially reflective mirror 425. When the white light is reflected off the partially reflective mirror 425, it is directed towards the table of the diamond 101. Due to the angular arrangement of the partially reflective mirror 425 the white light is directed towards the table of the diamond 101 along the same axis as the viewing axis 201, such that the light is propagated parallel to the viewing axis.

Within channels near to the plexi-glass device 429 are located four colour LEDs 435a, b, c and d, which form part of the apparatus that enables the diamond to be aligned. In this embodiment, the colour LEDs are red, green, yellow and blue respectively. The colour LEDs are also connected to a suitable power supply, which is operated by an ON/OFF switch (not shown). Note that in Figure 4, the colour LEDs 435c and d are not shown for clarity reasons. The LEDs are placed at 90° to each other such that the light is directed towards the centre of the plexi-glass 429 via channels 437.

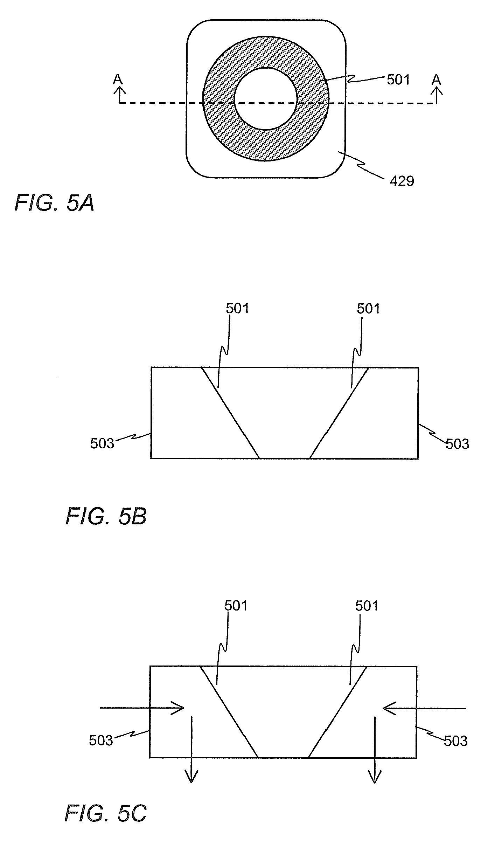

The plexi-glass device is now explained in more detail with reference to Figures 5a and 5b. Figure 5a shows a top view of a plexi-glass device according to an embodiment of this invention. The plexi-glass is made from polymethylmetacrylate (PMMA). The plexi-glass device 429 includes a conically shaped cut out portion with a matt surface 501. Figure 5b shows the plexi-glass device 429 from a cut out side view along lines A-A indicated in Figure 5A. It can be seen more clearly in Figure 5B that the cut out conical shape in the centre of the plexi-glass device includes the matt surface 501. Further, the outer edges of the plexi glass device comprise a transparent surface 503. Therefore, with this arrangement any light shining into the plexi glass device from the side passes through the transparent surface 501 until it arrives at the matt surface 501 where upon it is reflected off the matt surface at an angle of 90°, and out of the plexi-glass device as shown by the arrows in Figure 5C.

Referring back to Figure 4, the colour LEDs 435a-d thus direct coloured light towards the table of the diamond 101. In this manner, four different visually distinguishable zones, in this particular embodiment, coloured zones, are created around the area beneath the table of the diamond 101. Each zone covers a substantially 90° radial area about the viewing axis. The visually distinguishable zones are used to provide the user with an indication that misalignment has occurred.

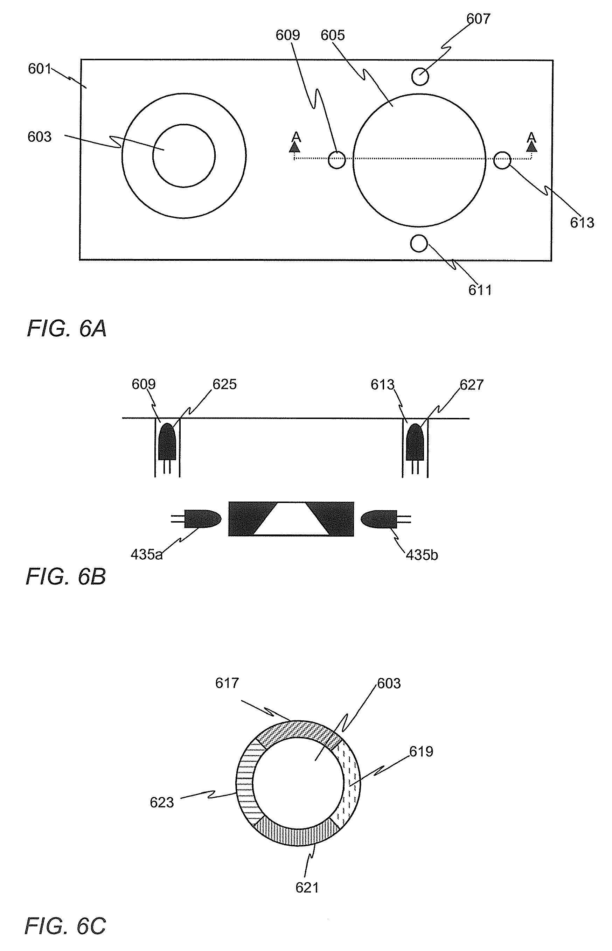

Figure 6A shows a top view of the gemstone viewing device. Figure 6B shows a partial cross section view along lines A-A shown in Figure 6A. The components indicated in Figures 6A and 6B also form part of the apparatus that enables the diamond to be aligned. Located on the top casing 601 of the device is the eyepiece 603, through which the user views the gemstone. The gemstone is placed on the platform 605. Located at 90° intervals around the platform 605 are four holes 607, 609, 611, 613. Directly beneath these holes, within the main body of the viewing apparatus, are located four colour adjustment indicator LEDs.

Referring to Figure 6B, only two of the four LEDs are shown for clarity purposes, the red indicator LED 625 and green indicator LED 627. Each of the four colour adjustment indicator LEDs is aligned vertically with its corresponding colour LED 435a-d. Thus, the red adjustment indicator LED 625 shines through the hole 609 above its respective red LED 435a. The same configuration applies to the green, blue and yellow adjustment indicator LEDs. The arrangement of the adjustment indicators provides a visual indication to the user of the apparatus of how to adjust the platform.

Thus, the combination of the misalignment indicators providing visually distinguishable zones and the adjustment indicators providing an indication of how to adjust the platform enables a user to adjust the position of a diamond's surface to cause retro reflection, as will be explained in detail below.

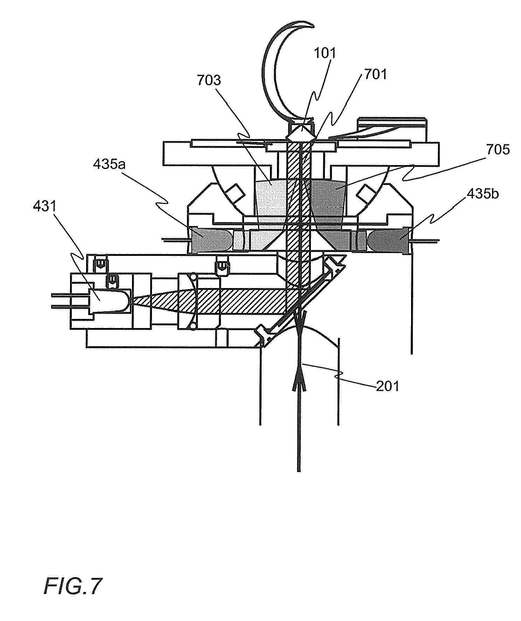

Figure 7 shows a close up view of the platform portion of the viewer with the white LED 431 and colour LEDS 435 a-d having been activated. It can be seen that along the viewing axis 201 the user of the viewing device views the table of the diamond 101. The white light emitted from the white LED 431 is reflected off the partially reflective mirror such that it is parallel with the viewing axis 201.

The colour LEDs 435a and 435b direct their coloured light via the plexi-glass device towards an area around the viewing axis and beneath the table of the diamond 101. That is, a red colour zone 703 is provided in one quadrant beneath the diamond 101. Further, a green colour zone 705 is provided in a second quadrant beneath the diamond 101. Also, two further colour zones are provided by the blue and yellow LEDs in two further quadrants beneath the diamond 101. For clarity reasons, the blue and yellow colour zones are not indicated.

When the platform upon which the diamond is sitting is adjusted such that the table of the diamond 101 is perpendicular to the viewing axis and the white light source, retro reflection occurs. The user will thus be able to see a bright white light reflected off the table of the diamond and also the darker ionised mark.

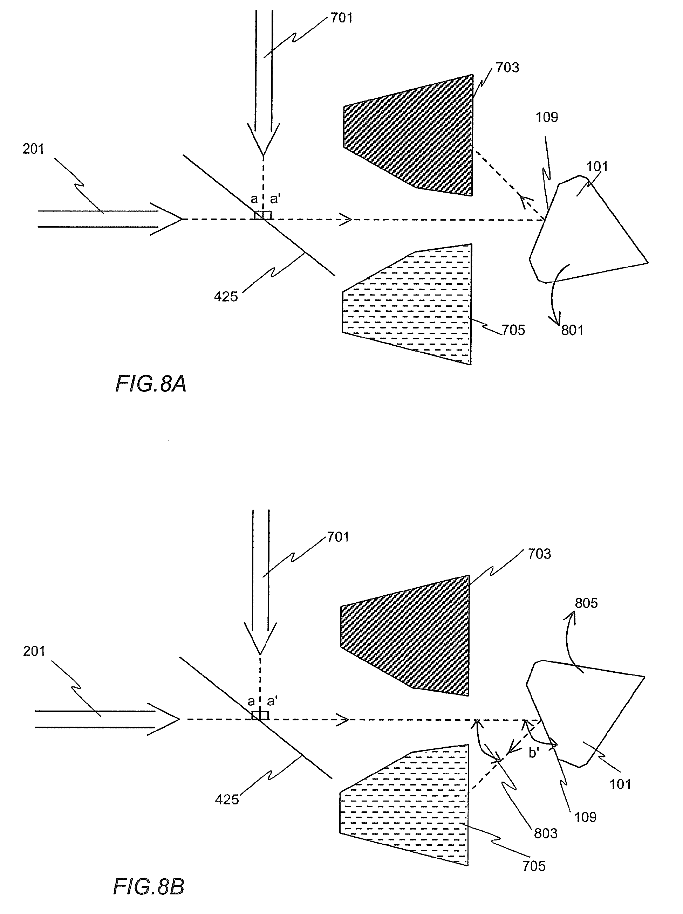

Now referring to Figures 8A and 8B, it will be explained how the user is directed to adjust the platform of the viewing device in order to cause retro reflection to occur.

Figure 8A shows an exaggerated side view of a diamond surface (table) that is not aligned perpendicular to the viewing axis 201 and the reflected white light. The white light 701 is directed towards the table 109 of the diamond 101 such that the white light propagation is parallel with the viewing axis 201. However, the user viewing the diamond along the viewing axis 201 would not see the light reflected off the table surface, as retro reflection does not occur as the table 109 of the diamond 101 is not perpendicular to the viewing axis 201. Rather, the user looking along the viewing axis 201 will see the red colour zone 703 reflected off the table 109 of the diamond 101, thus providing an indication that there is misalignment.

Referring to Figures 6A, 6B and 8A, the user will be able to determine in which direction the platform requires adjustment by associating the viewed colour zone reflected off the table with the matching colour indicator LED near the platform. Therefore, in this example, the user is directed towards tilting the platform of the viewing device down in the area where the red indicator LED 625 is located. The movement of the platform causes the diamond 101 to move in the direction as indicated by the arrow 801 in Figure 8A.

A further example of the gemstone viewing device providing an indication as to which direction to move the platform upon which the diamond 101 sits in order to cause retro reflection to occur is indicated in Figure 8B. In this figure it can be seen that the table 109 of the diamond 101 is also not perpendicular to the viewing axis 201. However, in this figure the table 109 directs the viewing axis towards the green colour zone 705. Therefore, the user looking through the eyepiece along the viewing axis 201 sees the green colour zone 705 reflected off the table of the diamond, which indicates to the user that the platform of the viewing device needs to be tilted down where the green coloured indicator LED 627 is positioned (Figure 6A, 6B).

In this manner, it is possible for a user to easily and quickly adjust the position of the diamond upon the platform in order for retro reflection to occur. Once retro reflection has occurred, the lateral position of the platform in the gemstone viewing apparatus may be altered in order to align the viewing axis 201 with the mark ionised on the table 109. Also, once retro reflection has occurred the surface of the table of the diamond may be brought into focus by adjusting the objective lens 423. The adjustment of the objective lens 423 may be carried out before or after retro reflection has occurred, as well as before or after the mark has been located.

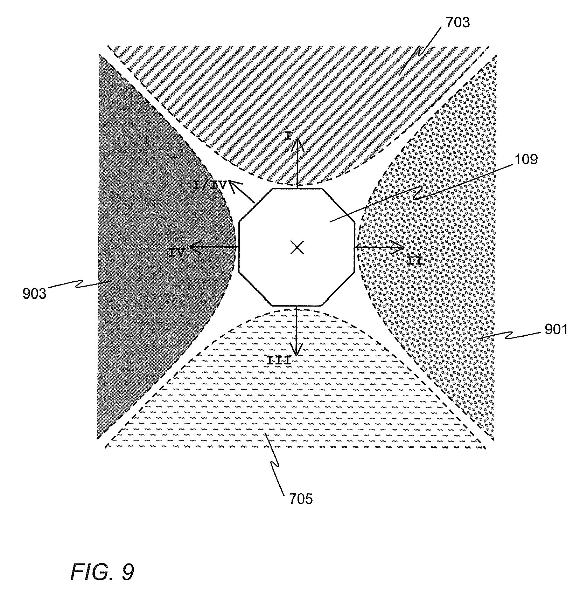

Figure 9 shows a view of the table of a diamond taken from the partially reflective mirror 425 when the table of a diamond is perpendicular to the viewing axis marked X. The four colour zones green, red, blue and yellow are indicated by the reference numerals 703, 705, 901 and 903.

It can be seen that if the table 109 of the diamond is tilted towards direction I (as indicated in the Figure) that the user will see the reflection of the green colour zone 703 off the table 109. Whereas, the user will see the blue colour zone 901 reflected off the table if the table is titled towards direction II (as indicated in the Figure). If the table 109 of the diamond is tilted towards direction III (as indicated in the Figure), the user will see the red colour zone 105 reflected off the table. If the user tilts the diamond table 109 in the direction IV (as indicated in the Figure), the user will see the reflected yellow colour zone 903 reflected off the table. If the table of the diamond 109 is tilted towards a junction between the colour zones 703 and 903 along the line I/IV (as indicated in the Figure), the user will see a mixture of the green colour zone 703 and the yellow colour zone 903 reflected off the table. Therefore, the user will see an indication that the platform needs to be tilted towards the intersection point of the green and yellow indicator LEDs on the platform.

This arrangement provides a user with an easy to use and quick guide on how to adjust a platform upon which a gemstone is located in order to cause retro reflection to occur and so enable a mark ionised on the gemstone to be viewed.

### FURTHER EMBODIMENTS

It will be understood that embodiments of the present invention are described herein by way of example only, and that various changes and modifications may be made without departing from the scope of the invention.

In an alternative arrangement, as shown in Figure 6C, colour LEDs are not used as adjustment indicators around the platform 605 to provide an indication to the user as to which side of the platform is to be adjusted to align the gemstone. Instead, coloured strips are placed internally within the eyepiece 603. When looking through the eyepiece 603, four separate quadrants of coloured area are visible, see reference numerals 617, 619, 621 and 623, which depict colours red, yellow, green and blue respectively. These colour indicators match up with the location of the respective coloured LEDs 435 a-d that provide the misalignment indication.

Further, it will also be understood that the adjustment indicators may be placed in any suitable position anywhere on the apparatus in order to aid the user in the adjustment of the platform. For example, the adjustment indicators may be placed on or around the platform area.

Further, it will also be understood that the adjustment indicators may comprise any suitable indication, as long as the adjustment indicator allows a user to associate the misalignment indicator with the adjustment indicator. For example, the words 'RED', 'GREEN', 'BLUE' and 'YELLOW' could be placed around the platform, or even within the eyepiece, to provide an indication of which way to adjust the platform based on a viewed reflected colour.

It will also be understood that the misalignment indicators could comprise three zones in order to implement the invention as described. That is, each of the zones provides a substantially 120° radial visually distinguishable zone around the viewing axis beneath the table 109 of the diamond. Also, any number over and above four visually distinguishable zones may be used to implement the invention. Also, it will be understood that different visually distinguishable zones other than those described above could be used. For example, visually distinguishable zones using patterns could be provided as an alternative to colour. One example is to use different patterned luminous or phosphorescent materials, of any suitable substance, arranged around the viewing axis in three or more zones to provide a reflected image, which can be matched up with an adjustment indicator associated with the same pattern.

Further, it will be understood that the alignment apparatus may comprise misalignment indicators other than coloured LEDs. That is, any suitable indicator may be provided around the viewing axis to show which way the surface of the gemstone is tilted, and so enable a user to adjust the platform to correct the misalignment. For example, different coloured luminescent or phosphorous materials could be place around the viewing axis beneath the platform so that the image reflected off the gemstone surface when misaligned is that of one of the colours from the luminous or phosphorescing material.

Further, it will be understood that the invention may be implemented to view any ionised or etched mark on any type of gemstone, where in the ionised or etched mark is located upon any surface of the gemstone.

Further, it will also be understood that the gemstone maybe affixed to the platform in any suitable manner. For example, either as a stand-alone gemstone, or when set within a piece of jewellery.

Further, it will also be understood that the eyepiece may be a moveable eyepiece to aid with focusing, and may also include a zoom function.

Further, it will also be understood that a camera could be utilised instead of an eyepiece, wherein the camera is connected to a monitor in order to aid the user when adjusting the position of the platform.

Further, it will also be understood that light sources other than LEDs could be used to implement this invention.

Further, the present invention is not limited to the arrangement as shown in the figures and described in the embodiments. Any suitable arrangement of the components could be used to implement the invention. For example, a smaller battery operated handheld version could be used wherein the re-directing mirrors along the viewing axis are not required. As a further example, although in the described embodiments above the platform is moved relative to the rest of the apparatus, a further embodiment may be provided wherein the platform is held in position while the viewing axis is moved relative to the platform in order to cause retro reflection to occur. In such an embodiment, the adjustment indicators are positioned relative to the viewing axis and not relative to the platform.

## Claims

1. Viewing apparatus for viewing a surface of a gemstone comprising an adjustable platform with a surface adapted to receive the gemstone, a viewing axis along which the gemstone is viewed, and a light source adapted to emit light substantially parallel to the viewing axis, the viewing apparatus
**characterised by**:
an alignment means comprising a plurality of misalignment indicators, the misalignment indicators arranged to provide three or more visually distinguishable zones around the viewing axis,
the alignment means further comprising three or more adjustment indicators positioned relative to the platform and the misalignment indicators,
wherein the adjustment indicators are associated with the visually distinguishable zones such that an image viewed along the viewing axis of one of the zones reflected off the surface of the gemstone provides an indication of which way to adjust the platform and the viewing axis relative to each other to make the surface of the gemstone and the viewing axis perpendicular to each other.

2. The viewing apparatus of claim 1, wherein the visually distinguishable zones are coloured zones.

3. The viewing apparatus of claim 1, wherein the visually distinguishable zones are patterned zones.

4. The viewing apparatus of claim 2, wherein the misalignment indicators are coloured light emitting diodes arranged to emit coloured light around the viewing axis.

5. The viewing apparatus of claim 1, wherein each misalignment indicator is a visually distinctive luminous or phosphorescent material arranged around the viewing axis.

6. The viewing apparatus of claim 5, wherein each misalignment indicator is a different colour.

7. The viewing apparatus of claim 5, wherein each misalignment indicator has a different pattern.

8. The viewing apparatus of claim 1 wherein the misalignment indicators provide four zones, each zone covering a substantially 90° radial area around the viewing axis.

9. The viewing apparatus of claim 1 wherein the misalignment indicators provide three zones, each zone covering a substantially 120° radial area around the viewing axis.

10. The viewing apparatus of claim 1 wherein each of the adjustment indicators is positioned to correspond with the position of one of the zones.

11. The viewing apparatus of claim 10, wherein the adjustment indicators are coloured light emitting diodes.

12. The viewing apparatus of claim 11, wherein the light emitting diodes are positioned around the platform and arranged to emit light generally towards the platform.

13. The viewing apparatus of claim 11, wherein the colours of the coloured light emitting diodes correspond to the coloured zones.

14. The viewing apparatus of claim 10 wherein the adjustment indicators are either coloured or patterned strips.

15. The viewing apparatus of claim 14 further comprising an eyepiece adapted to view along the viewing axis, wherein the adjustment indicators are positioned within the eyepiece.

16. The viewing apparatus of claim 14, wherein the adjustment indicators are positioned around the platform.

17. A method of viewing a mark on a surface of a gemstone, comprising the steps of:
placing the gemstone on a surface of a platform,
viewing the gemstone along a viewing axis, the method **characterised by** the steps of:
viewing along the viewing axis a visually distinguishable zone reflecting off the surface of the gemstone, the visually distinguishable zone being associated with an adjustment indicator, and
adjusting the platform and the viewing axis relative to each other depending on the associated adjustment indicator.

18. The method clam 17, wherein the visually distinguishable zones are coloured zones.

19. The method claim 17, wherein the visually distinguishable zones are patterned zones.

20. The method of claim 17 wherein four zones are provided, each zone covering a substantially 90° radial area around the viewing axis.

21. The method of claim 17 wherein three zones are provided, each zone covering a substantially 120° radial area around the viewing axis.

22. The method of claim 17, wherein each of the adjustment indicators is positioned to correspond with the position of one of the zones.

23. The method of claim 17, wherein the adjustment indicators are positioned around the platform.

24. The method of claim 17 wherein the gemstone is viewed through an eyepiece and the adjustment indicators are positioned within the eyepiece.

25. An apparatus for viewing a surface of a gemstone wherein alignment means is provided to give an indication of how to adjust the position of the gemstone to view the surface.

## Amended claims

### Amended claims in accordance with Rule 86(2) EPC.

**1.** Viewing apparatus for viewing a surface of a gemstone (101) comprising an adjustable platform (402) with a surface (403) adapted to receive the gemstone (101), a viewing axis (201) along which the gemstone (101) is viewed, and a light source (431) adapted to emit light substantially parallel to the viewing axis (201), the viewing apparatus **characterised by**:
an alignment means (607, 609, 611, 613, 625, 627, 435a-d) adapted to provide an indication of which direction to adjust the platform (402) and the viewing axis (201) relative to each other to make a surface of the gemstone (101) and the viewing axis (201) perpendicular to each other.

**2.** The viewing apparatus of claim 1, wherein the alignment means comprises a plurality of misalignment indicators (435a-d), the misalignment indicators (435a-d) arranged to provide three or more visually distinguishable zones (703, 705, 901, 903) around the viewing axis (201),
the alignment means further comprising three or more adjustment indicators (607, 609, 611, 613) positioned relative to the platform (402) and the misalignment indicators (435a-d),
wherein the adjustment indicators (607, 609, 611, 613) are associated with the visually distinguishable zones (703, 705, 901, 903) such that an image viewed along the viewing axis (201) of one of the zones reflected off the surface of the gemstone provides said indication.

**3.** The viewing apparatus of claim 1, wherein the visually distinguishable zones (703, 705, 901, 903) are coloured zones.

**4.** The viewing apparatus of claim 1, wherein the visually distinguishable zones (703, 705, 901, 903) are patterned zones.

**5.** The viewing apparatus of claim 3, wherein the misalignment indicators (435a-d) are coloured light emitting diodes arranged to emit coloured light around the viewing axis (201).

**6.** The viewing apparatus of claim 1, wherein each misalignment indicator is a visually distinctive luminous or phosphorescent material arranged around the viewing axis.

**7.** The viewing apparatus of claim 6, wherein each misalignment indicator (435a-d) is a different colour.

**8.** The viewing apparatus of claim 6, wherein each misalignment indicator (435a-d) has a different pattern.

**9.** The viewing apparatus of claim 1 wherein the misalignment indicators (435a-d) provide four zones, each zone covering a substantially 90° radial area around the viewing axis (201).

**10.** The viewing apparatus of claim 1 wherein the misalignment indicators (435a-d) provide three zones, each zone covering a substantially 120° radial area around the viewing axis.

**11.** The viewing apparatus of claim 1 wherein each of the adjustment indicators (607, 609, 611, 613) is positioned to correspond with the position of one of the zones.

**12.** The viewing apparatus of claim 11, wherein the adjustment indicators (607, 609, 611, 613) are coloured light emitting diodes.

**13.** The viewing apparatus of claim 12, wherein the light emitting diodes (607, 609, 611, 613) are positioned around the platform (402) and arranged to emit light generally towards the platform (402).

**14.** The viewing apparatus of claim 12, wherein the colours of the coloured light emitting diodes (607, 609, 611, 613) correspond to the coloured zones.

**15.** The viewing apparatus of claim 11 wherein the adjustment indicators are either coloured or patterned strips.

**16.** The viewing apparatus of claim 15 further comprising an eyepiece (415) adapted to view along the viewing axis (201), wherein the adjustment indicators (607, 609, 611, 613) are positioned within the eyepiece (415).

**17.** The viewing apparatus of claim 15, wherein the adjustment indicators (607, 609, 611, 613) are positioned around the platform (402).

**18.** A method of viewing a mark on a surface of a gemstone, comprising the steps of:
placing the gemstone on a surface of a platform,
viewing the gemstone along a viewing axis, the method **characterised by** the steps of:
viewing along the viewing axis a visually distinguishable zone reflecting off the surface of the gemstone, the visually distinguishable zone being associated with an adjustment indicator, and
adjusting the platform and the viewing axis relative to each other depending on the associated adjustment indicator.

**19.** The method clam 18, wherein the visually distinguishable zones are coloured zones.

**20.** The method claim 18, wherein the visually distinguishable zones are patterned zones.

**21.** The method of claim 18 wherein four zones are provided, each zone covering a substantially 90° radial area around the viewing axis.

**22.** The method of claim 18 wherein three zones are provided, each zone covering a substantially 120° radial area around the viewing axis.

**23.** The method of claim 18, wherein each of the adjustment indicators is positioned to correspond with the position of one of the zones.

**24.** The method of claim 18, wherein the adjustment indicators are positioned around the platform.

**25.** The method of claim 18 wherein the gemstone is viewed through an eyepiece and the adjustment indicators are positioned within the eyepiece.
